# EUROPÄISCHE PATENTANMELDUNG

(11) **EP 1 505 079 A2**
(43) Veröffentlichungstag der Anmeldung: **09.02.2005**
(21) Anmeldenummer: 04023290.2
(22) Anmeldetag: 22.12.2000
(51) Int. Cl.: C08B 37/10, A61K 31/727

(54) **Heparin mit mittlerer Molmasse von 10 bis 11,5 kD**

(30) Priorität: 10.01.2000 DE 10000602
(62) Teilanmeldung aus: 00990588.6
(71) Anmelder: Prof. Welzel, Dieter, 90419 Nürnberg (DE)
(72) Erfinder: Prof. Welzel, Dieter, 90419 Nürnberg (DE)
(74) Vertreter: Jönsson, Hans-Peter, Dr.

(57) **Zusammenfassung**

Gegenstand der Erfindung ist ein neues Heparin, das eine mittlere Molmasse im Bereich von 10 bis 11,5 kd aufweist, insbesondere eine mittlere Molmasse von 10,5 kd. Dieses Heparin ist durch kontrollierte Depolymerisierung von unfraktioniertem Heparin und nachfolgender Molekularfiltration erhältlich und wird zur Herstellung eines Arzneimittels zur Prophylaxe und Therapie thromboembolytischer Prozesse und, unter anderem, zur Gerinnungshemmung in extrakorporalen Kreisläufen verwendet.

## Beschreibung

Die vorliegende Erfindung betrifft allgemein ein neues Heparin mit mittlerer Molmasse, dessen Herstellung und Verwendung.

Heparin wurde 1916 von MacLean entdeckt und wird bereits seit über 60 Jahren in der Medizin eingesetzt. Haupteinsatzgebiet ist dabei die antithrombotische Prophylaxe und Therapie. Mittlerweile wird Heparin in der Medizin in zwei unterschiedlichen Formen eingesetzt. Hierbei ist zunächst das unfraktionierte Heparin (UFH) zu nennen, das großtechnisch aus Lunge, Leber oder Darmschleimhaut von Rindern und Schweinen gewonnen wird und nach Proteolyse, Abtrennung unerwünschter Begleitstoffe, wie z.B. Fett, Proteine, und Bleichung erhalten wird. Als zweites sind niedermolekulare Heparine (NMH) zu nennen, die durch Depolymerisierung von UFH gewonnen werden.

Der wesentliche Unterschied dieser beiden Heparine bei der Anwendung liegt in der unterschiedlichen Bioverfügbarkeit. Diese beträgt nach subkutaner Injektion bei UFH ca. 10-20 % der applizierten Dosis, wobei die Bioverfügbarkeit der NMH bei 90 % liegt, (Kandotas, R.J., Heparin Pharmacokinectics and Pharmacodynamics, Clinical Pharmacokinectics, 22(5):359-374, 1992).

In der nachfolgenden Tabelle 1 sind wesentliche Charakteristika von UFH und NHM aufgeführt und einander gegenübergestellt. (Hirsh, J. und Levine M. N., "Low molecular weight heparine", Blood 79(1 ):1-17, 1992).

**Tabelle 1**

| | UFH | NMH |
|---|---|---|
| Molekulargewicht (in d) | 3.000-30.000 | 2000-8.000 |
| mittleres MG (in d) | 13.000 | 5.000 |
| anti-Xa/anti-IIa-Aktivität | 1:1 | 2:1 - 5:1 |
| Neutralisation durch PF 4 ^{*} | stark | schwach |
| Bindung an Proteine | hoch | niedrig |
| Bindung an Endothelzellen | ja | schwach |
| dosisabhängige Clearance | ja | nein |
| Bioverfügbarkeit (s.c.) | 10-25 % | ∼ 90 % |
| Halbwertszeiten (t½) | 1 h (i.v.) | 2 h (i.v.) |
| | 1 - 2 h (s.c.) | 3 - 6 h (s.c.) |
| Elimination | biophasisch | renal |
| Laborkontrollen | erforderlich | nicht erforderlich |

| | | |
|---|---|---|
| ^{*} PF 4 = Plasmaprotein mit Namen Plättchenfaktor 4 | | |

In der klinischen Anwendung werden UFH und NMH im wesentlichen zur Prophylaxe und Therapie thromboembolischer Erkrankungen eingesetzt, wobei NMH erst seit kürzerer Zeit vermehrt Einsatz gefunden hat. Insgesamt konnte das peri- und postoperative Thromboserisiko von 50 bis 60 % durch den Einsatz dieser Heparine auf etwa 15 bis 30 % reduziert werden (Harenberg, J., Haas, S. und Breddin, K.H., "Prophylaxe der venösen Thrombose" in Müller-Berghaus, G. Pötsch (Hrsg.), "Hämostaseologie", Springer Verlag, Berlin-Heidelberg, Seiten 564 - 580, 1998). Die erreichte Senkung des peri- und postoperativen Thromboserisikos auf 15 bis 30 % zeigt, daß Bedarf an einer weiteren Senkung des Thromboserisikos besteht, weshalb die Forschung um die Entwicklung verbesserter Alternativen bemüht ist.

Die Entwicklung von Hirudin, einem direkten Thrombininhibitor, brachte hier eine gewisse Verbesserung, weil ein weiter reichender Schutz erreichbar war. Der Einsatz von Hirudin beschränkt sich jedoch auf besondere Problemfälle, z.B. eine Heparin-Unverträglichkeit, Behandlung einer heparininduzierten Thrombozytopenie (HIT), weil für Hirudin kein Antidot existiert und somit die Gefahr unkontrollierbarer Blutungen besteht.

NM-Heparine werden trotz aller Verschiedenheit der einzelnen Präparate als klinisch äquivalent eingestuft und in Metaanalysen regelmäßig zusammengefaßt. Sie übertreffen UFH in der Orthopädie bzw. im operativen Hüftgelenkersatz jedoch nur in punkto Wirksamkeit, Hinsichtlich der Verträglichkeit bestehen keine Unterschiede. In dem dominierenden Anwendungsbereich der Allgemeinchirurgie erweisen sich NMH und Standardheparin als durchgängig gleichwertig. Dies trifft zumindest hinsichtlich der Hauptkriterien Thromboseschutz und Blutungsneigung zu.

Die Ebenbürdigkeit der einzelnen NM-Heparine in der Klinik kontrastiert zu den Unterschieden im mittleren Molekulargewicht, dem Molekulargewichtsspektrum, den Prozenten aus anti-FXa- und anti-Flla-Aktivität sowie dem Einfluß auf die APTT, eine Gerinnungszeit, die die Hemmung der endogenen Thrombinaktivität (durch Heparin) signalisiert. Die Liste der Unterschiede umfaßt ferner die profibrinolytische Wirkung, die Freisetzung von TFPI (tissue factor pathway inhibitor), die Beeinflussung der Plättchenfunktion etc..

Parallel zur labormedizinischen Vielfalt der einzelnen Produkte gibt es keine Möglichkeit für eine übergeordnete klinische Effektivitätskontrolle, die die Entwicklung eines neuen Heparins determinieren könnte.

Zusammenfassend gibt es daher keinerlei Anhaltspunkte, wie man Heparine in punkto therapeutischer Breite als Verhältnis von Wirksamkeit und Verträglichkeit optimieren könnte.

Es ist daher Aufgabe der vorliegenden Erfindung einen Wirkstoff mit antikoagulatorischer Wirkung anzugeben, und somit den Stand der Technik zumindest um einen weiteren Wirkstoff zu bereichern, wobei dieser Wirkstoff zumindest einen Teil der aus dem Stand der Technik bekannten Nachteile überwinden soll.

Es ist weiterhin Aufgabe der vorliegenden Erfindung eine Verwendung für den erfindungsgemäßen Wirkstoff anzugeben.

Die vorliegende Aufgabe wird durch ein Heparin mit den Merkmalen des beigefügten Anspruchs 1 gelöst. Ebenso wird die vorliegende Aufgabe durch die Verwendung eines solchen Heparins gemäß dem beigefügten Anspruch 3 gelöst.

Überraschend ist nun gefunden worden, daß Heparin mit einer mittleren Molmasse von 10 bis 11,5 kd, besonders bevorzugt ein Heparin mit einer mittleren Molmasse von 10,5 kd, ein Wirkungsspektrum zeigt, daß denen von UFH und NMH deutlich verschieden ist.

Grundsätzlich wäre aufgrund der Eignung von NMH und UFH zu erwarten gewesen, daß das erfindungsgemäße Heparin mit mittlerer Molmasse ein Wirkungsspektrum zeigt, das in etwa einem gemittelten Wirkungsspektrum von UFH und NMH entspricht.

Durch die vorliegende Erfindung wird daher insbesondere auch aufgrund der Tatsache, daß bei höherer Wirksamkeit keine gegenüber den bekannten Heparinen erhöhte Blutungsneigung auftritt, ein lang anhaltendes Bedürfnis nach einer Verbesserung der Prophylaxe und Therapie thromboembolytischer Prozesse Rechnung getragen.

Die Bewertung des erfindungsgemäßen Heparins erfolgte durch Vergleich mit Enoxaparin dem international führenden NMH. Da NMH allgemein als wenigstens genauso wirksam und tolerierbar wie UFH angesehen werden, können die aus dem Vergleich zwischen dem erfindungsgemäßen Heparin und Enoxaparin auf bestimmte Weise auch auf UFH ausgedehnt werden.

Zur näheren Erläuterung wird die Erfindung nachfolgend in Form von Beispielen und Vergleichen mit dem aus dem Stand der Technik bekannten Heparinen näher erläutert, wobei die nachfolgenden Ausführungen ausschließlich zur näheren Erläuterung der Erfindung dienen sollen und nicht etwa zu deren Beschränkung.

### Es zeigen:

- Figur 1: eine Darstellung der Molmassenverteilung des erfindungsgemäßen Heparins (HMM), von Enoxaparin (NMH) und von Liquemin® (UFH) in einem GPC-Elutionsprofil,
- Figur 2a: eine Darstellung der APTT-Aktivität jeweils des erfindungsgemäßen Heparins (HMM), von Enoxaparin (NMH) und von Liquemin® (UFH),
- Figur 2b: eine Darstellung des anti-Faktor-Xa-Aktivität der drei Heparine,
- Figur 2c: eine Darstellung der Antithrombinaktivität der drei Heparine,
- Figur 3a: eine Darstellung der AUC₀₋₂₄ₕ-Werte der drei Heparine,
- Figur 3b: eine Figur 3a entsprechende Darstellung der anti-FXa-Aktivität der drei Heparine,
- Figur 3c: eine den Figuren 3a und 3b entsprechende Darstellung der anti-FIIA-Aktivität der drei untersuchten Heparine,
- Figuren 4a bis 4c: eine Darstellung der dosisnormierten Aktivitäten entsprechend den Figuren 3a bis c der drei untersuchten Heparine,
- Figur 5a: eine zeitabhängige Darstellung der Konzentration an Gesamt TFPI-Antigen jeweils nach Gabe eines Heparins (durchgezogene Linie HMM; gepunktete Linie NMH (Enoxaparin); gestrichelte Linie UFH (Liquemin®)),
- Figur 5b: eine Darstellung gemäß Figur 5a für das freie TFPI-Antigen,
- Figuren 6a bis 6d: eine Darstellung der AUC0-24h-Werte für die drei untersuchten Heparine, berechnet aus der Freisetzung von Gesamt-TFPI-Antigen sowie von freiem TFPI-Antigen.
- Figur 7: Vergleich der antithrombotischen Wirkungen von Enoxaparin und dem erfindungsgemäßen Heparin mittlerer Molmasse im Tierversuch (Kaninchen),
- Figur 8: Vergleich der Blutungsdauer bei Verabreichung von Enoxaparin bzw. dem erfindungsgemäßen Heparin mittlerer Molmasse im Tiermodell (Kaninchen).

Das erfindungsgemäße Heparin wurde mittels kontrollierter Depolymerisation hergestellt und weiter mittels molekularer Filtertechniken aufgearbeitet. Das so erhaltene Heparin wies folgendes HPLC-Profil auf:

| | |
|---|---|
| zahlengemittelte Molmasse | 10.513 |
| Gewichtsgemittelte Molmasse | 10.819 |
| Z-Mittel | 11.233 |
| Viskositätsmittel | 10.819 |
| Z+1-Mittel | 11.815 |
| Innere Viskosität | 0,0000 |
| Peak-Molmasse | 10.236 |
| Dispersity | 1,029066 |
| Z-Mittel/Gewichtsmittel | 1,038271 |
| Z+1-Mittel/Gewichtsmittel | 1,092039 |

Zur weiteren Charakterisierung und zur Demonstration der überragenden Eigenschaften des erfindungsgemäßen Heparins wurde das erfindungsgemäße Heparin mit einem unfraktionierten Heparin, Liquemin®, und einem niedermolekularen Heparin, Enoxaparin, verglichen.

Das erfindungsgemäße Heparin mit mittlerer Molmasse wird in dieser Beschreibung auch mit "HMM" bezeichnet, Enoxaparin als Vertreter der niedermolularen Heparine mit "NMH" und Liquemin@ als Vertreter der unfraktionierten Heparine mit "UFH".

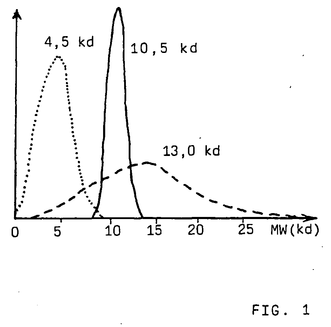

Hinsichtlich ihrer mittleren Molmassen und ihrer Molmassenverteilung unterscheiden sich das erfindungsgemäße Heparin UFH und NMH deutlich voneinander, wie es aus der Figur 1 hervorgeht. Aus Figur 1, die ein GPC-Elutionsprofil der drei genannten Heparine darstellt ist eindeutig zu entnehmen, daß das UFH eine mittlere Molmasse von 13,0 kd bei gleichzeitig sehr breiter Molmassenverteilung besitzt. Das NMH besitzt eine mittlere Molmasse von 4,5 kd und eine deutlich engere Molmassenverteilung. Das erfindungsgemäße Heparin hingegen besitzt eine mittlere Molmasse von 10,5 kd, wobei es gleichzeitig die engste Molmassenverteilung der drei unterschiedlichen Heparine aufweist.

Der engen Molmassenverteilung des erfindungsgemäßen Heparins kommt dabei eine besondere Bedeutung zu, weil hierauf das einzigartige und überraschende pharmakologische Wirkungsspektrum des Heparins der vorliegenden Erfindung zurückzuführen ist.

Bedeutende und unerwartende Unterschiede ergeben sich aus einem Vergleich der *in vitro*-Aktivitäten der drei Heparine. Hierbei wird auf das international standardisierte Maßsystem der anti-Xa-Aktivität zurückgegriffen, bei dem die anti-Xa-Aktivität auf 1 mg des Wirkstoffs bezogen ist. Zum Vergleich wurden auch Messungen der anti-IIa-Aktivität durchgeführt. Die Ergebnisse dieser Untersuchung sind in der nachfolgenden Tabelle 2 aufgeführt.

**Tabelle 2**

| | **aXa i.E./mg** | **aIIa i.E./mg** | **aXa/aIIa-Verhältnis** | **aIIa/aXa-Verhältnis** |
|---|---|---|---|---|
| HMM | 174,9¹ | 170,0¹ | 1,03 | 0,97 |
| Liquemin® (UFH) | 159,0² | 159,0² | 1,00 | 1,00 |
| Enoxaparin (NMH) | 100,0² | 26,3² | 3,80 | 0,26 |

| | | | | |
|---|---|---|---|---|
| ¹ bestimmt nach dem 1. internationalen Standard für NMH | | | | |
| ² bestimmt nach dem 4. internationalen Standard für UFH | | | | |

Hierbei ist besonders überraschend, daß das erfindungsgemäße Heparin, HMM, gegenüber den beiden Vergleichswirkstoffen, die jeweils ein UFH und NMH repräsentieren, sowohl hinsichtlich der anti-Xa-Aktivität als auch hinsichtlich der anti-IIa-Aktivität eine deutliche Steigerung der Aktivität aufweist. Dies war auf keinen Fall zu erwarten. Bestenfalls hätte man annehmen können, daß die anti-Faktor Xa- und Antithrombin-Aktivitäten des erfindungsgemäßen Heparins eine Mittelstellung zwischen UFH und NMH einnehmen.

Eine randomisierte, multiple cross-over Doppelblinduntersuchung wurde an 16 gesunden männlichen Personen (Alter: 18-32 Jahre, Gewicht: 64-98 kg) zur pharmakokinetischen und hämostasiologischen Charakterisierung des erfindungsgemäßen Heparins (HMM) im Vergleich zu Liquemin® (UFH) und Enoxaparin (NH) durchgeführt. Jede der Untersuchungspersonen erhielt eines der drei Heparine als eine Einzeldosis von 9.000 anti-Xa i.E. in einem einwöchigen Intervall. Blut wurde vor jeder Injektion und 0,5, 1, 2, 3, 4, 5, 8, 10 und 24 Stunden nach jeder Injektion genommen und mit Citrat gepuffert. Die ersten zwei Milliliter des Blutes wurden verworfen und das Plasma wurde gemäß allgemeinen Richtlinien erhalten (Witt, I. Beeser, H. und Müller-Berghaus, G. "Minimalanforderungen zur Gewinnung von Citratplasma für hämostasiologische Analysen", Lab. Med. Seiten 143-145, 1995). Die erhaltenen Proben wurden in aliquote Portionen unterteilt und unmittelbar danach mit flüssigem Stickstoff eingefroren und bei -70 °C gelagert, bis die Messungen durchgeführt wurden.

Die dann an den Blutproben durchgeführten *ex vivo*-Untersuchungen wurden entsprechend den nachfolgend beschriebenen Bedingungen durchgeführt.

Hierbei ist festzustellen, daß es sich bei den nachfolgend vorgestellten Ergebnissen um die Ergebnisse der *in vivo*-Wirkungen des erfindungsgemäßen Heparins (HMM) im Vergleich zu UFH und NMH handelt, die *ex vivo* untersucht wurden.

Die antikoagulatorische Aktivität der Plasmaproben wurde durch Bestimmung der Gerinnungszeit gemäß APTT- (activated partial thrombin time) Test ermittelt (APTT-Micro Kieselgur, Instrumentation Laboratory), wobei die Inhibierung von Faktor Xa sowie der Thrombinaktivität auf einem ACL 3000-Modell (Instrumentation Laboratory) bestimmt wurde. Bei dieser Untersuchung handelt es sich um die Bestimmung eines Gerinnungsparameters, der allgemein zur Bestimmung von antikoagulatorischen Wirkungen herangezogen wird. Es wurden allgemein zwei Werte bestimmt. Wenn die Abweichung mehr als 3% betrug, wurde die Messung wiederholt. Die Gültigkeit wurde durch parallel durchgeführte Kontrollen mit Vergleichsplasma bestimmt, dem 4. internationalen Standard für UHF und dem 1. internationalen Standard für NMH.

Für die Bestimmung der anti-Xa- und anti-IIa-Aktivität wurde das Plasma mit Puffem zur Heparinbestimmung (Chromogenix) verdünnt und mit menschlichem AT (Chromogenix) ergänzt. Nach Inkubation mit Faktor Xa oder Thrombin (Chromogenix) wurde die verbleibende Aktivität jeweils mittels der Reaktion der chromogenen Substrate S2222 und S2238 gemessen.

Die Gesamt- und freien TFPI-Antigenkonzentrationen in den Plasmaproben wurden mittels der spezifischen ELISAs "Asserachrom® Free TPFI" und "Asserachrom® Total TFPI" (Diagnostica Stago) gemessen. Mit F(ab')2-Fragmenten von einem für TFPI spezifischen Antikörper (TT4E2) beschichtete ELISA-Platten wurden für die TFPI-Messung verwendet. Die monoklonalen, Peroxidase-gekoppelten Nachweisantikörper waren entweder für gesamtes TFPI spezifisch (2C6) oder für freies TFPI (H65).

Die aus den Untersuchungen gewonnenen Kurven der Verlängerung der Gerinnungszeit, der Inhibierung von Faktor Xa und Thrombin (Faktor IIa) und den Gesamt- und freien TFPI-Konzentrationen der Einzelpersonen wurden verwendet, um aus ihnen die folgenden pharmacokinetischen Paramter zu bestimmen:
- AUC₀₋₂₄ₕ[s*h,%Inh.*h oder (ng/ml)*h] = Fläche unter der Kurve
   = (area under the curve)
- cₘₐₓ[S, inh.% oder ng/ml] = Aktivitätsmaximum (Verlängerung
   der Gerinnungszeit, Inhibierung oder
   Konzentration)
- tₘₐₓ[Min.] = Zeit des Aktivitätsmaximums
- t1/2_{Invasion}[min.] = Invasions-Halbwertszeit
- t1/2Elimination[min.] = Eliminations-Halbwertszeit

Die Werte für cₘₐₓ und tₘₐₓ wurden direkt aus den einzelnen Kurven bestimmt. AUC wurde mittels der Trapezregel zwischen t=0 h und t=24 h berechnet.

Außer einem Basislinienwert von 0 ist Voraussetzung der Bestimmung von t1/2_{Invasion} und t1/2 _{Elimination} , daß Invasion und Elimination in Bezug auf die Zeit exponentiell verlaufen. Zur Überprüfung dieser Annahmen wurden die gemessenen Daten einer Biexponenzialfunktion angepaßt (Bateman Funktion, erweitert um einen log-Ausdruck). Ein Vergleich der angepaßten und gemessenen Kurven zeigte eine sehr gute Übereinstimmung in den meisten Fällen, so daß die Kinetik von Heparin und TFPI gut durch die Bateman Funktion beschrieben werden kann. Die Werte von t1/2_{Invasion} und t1/2 _{Elimination} wurden aus den konstanten der Bateman-Funktion für die Invasion und Elimination (kᵢₙᵥ, Kₑₗ) berechnet. Sämtliche Werte wurden als Mittelwert ± Standardabweichung bestimmt. Der paarweise Vergleich sämtlicher Parameter zwischen den drei Heparinen wurde durch Gegenüberstellung unter Verwendung eines Signifikanzniveaus (a) von 0,05 gemacht. Unterschiede wurden als statisch signifikant angesehen, wenn p < 0,05 war. Die Mittelwerte der pharmacokinetischen Parameter wurden nachfolgend einem allgemeinen Linearmodell der Varianzanalyse unterworfen (Null-Hypothese:es existiert kein Unterschied jeweils zwischen dem erfindungsgemäßen Heparin und Liquemin® und Enoxaparin).

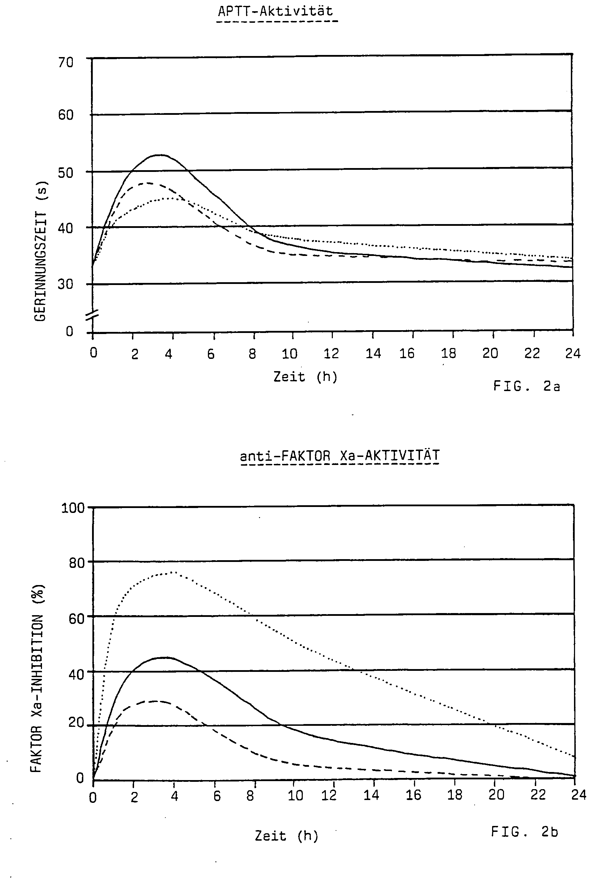

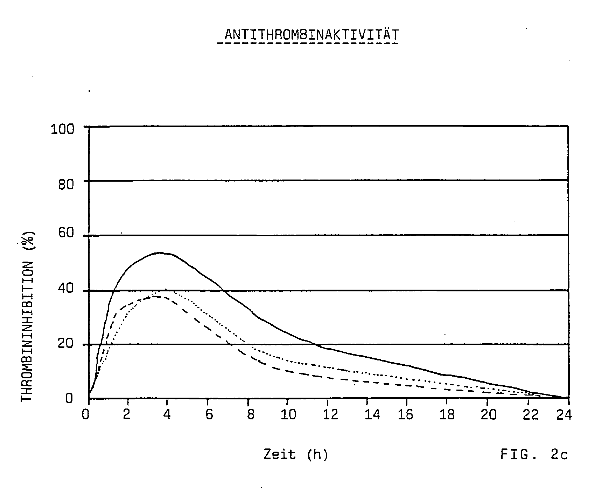

Als Ergebnis ist festzustellen, daß die Kurven der Wirkungen der drei unterschiedlichen Heparine klare und deutliche Unterschiede in den verschiedenen Untersuchungen zeigen, wie aus den Figuren 2a bis c hervorgeht.

Dabei wird deutlich, daß das erfindungsgemäße Heparin die höchste Aktivität in dem APTT-Test und dem anti-Flla-Assay zeigt und sich insbesondere in seiner anti-FIIa-Aktivität von den beiden anderen Heparinen auf überraschende Weise unterscheidet. Einzig das anti-FXa-Assay zeigt ein erwartetes Ergebnis. Hier nimmt das erfindungsgemäße Heparin in etwa eine Mittelstellung zwischen Enoxaparin und Liquemin® als Beispiel für NMH und UFH ein.

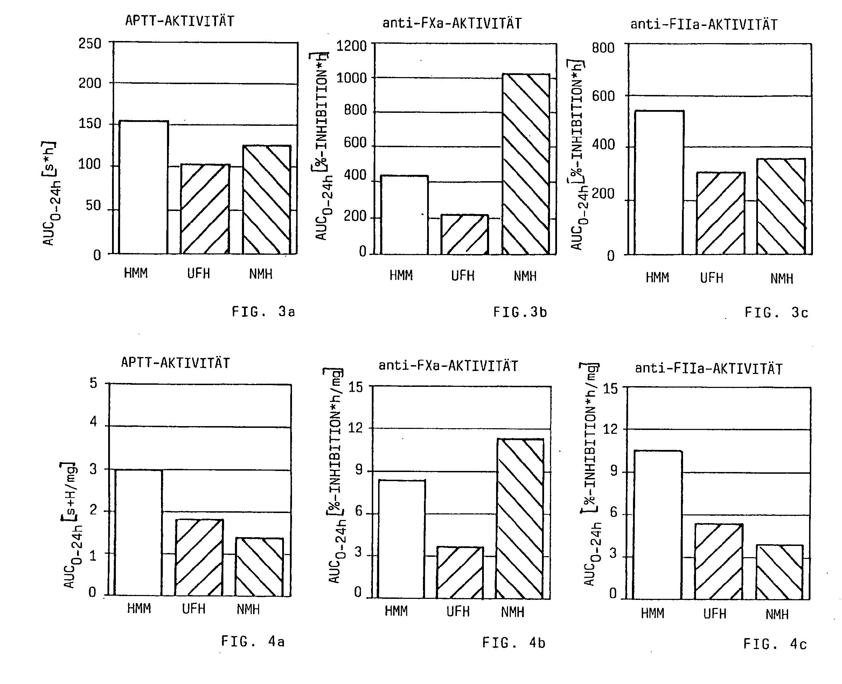

Das unterschiedliche antikoagulatorische Potential der untersuchten drei Heparine wird aus den AUC₀₋₂₄ₕ-Daten des APTT-Tests, des anti-FXa- und des anti-FIIa-Assays deutlich, die in den Figuren 3a bis c jeweils für die 3 untersuchten Heparine dargestellt sind. Die Werte resultieren dabei aus der Wirkung des jeweiligen Heparins in den einzelnen Tests, d.h. der Verlängerung der Gerinnungszeit sowie jeweils der Inhibierung von Faktor Xa und Thrombin. Dabei zeigt sich, daß die drei Heparine sich nicht nur signifikant in ihrer Pharmacokinetik unterscheiden, sondern auch in ihrer Pharmacodynamik (z.B. der Größenordnung der anti-FXa-Aktivität im Vergleich mit der anti-FIIA-Aktivität).

Dabei fällt die jeweils bis zu zweifache Aktivität des erfindungsgemäßen Heparins im Vergleich zu Liquemin® in allen drei Assays auf. Hieraus läßt sich schließen, daß die Bioverfügbarkeit des erfindungsgemäßen Heparins, das nicht die für unfraktionierte Heparine typischen Moleküle mit hoher Molmasse enthält, nach subkutaner Administration signifikant größer ist. Auf der anderen Seite zeigt ein Vergleich des erfindungsgemäßen Heparins mit Enoxaparin, daß das erfindungsgemäße Heparin nicht zu den Heparinen mit niederer Molmasse gehört. Während die anti-FaktorXa-Aktivität von Enoxaparin mehr als doppelt so hoch wie die des erfindungsgemäßen Heparins ist, ist dessen Antithrombinaktivität nur etwa 66 % von der des erfindungsgemäßen Heparins. Hieraus läßt sich schließen, daß die Bioverfügbarkeit von Enoxaparin wirklich höher ist, die thrombininhibierende Wirkung, welche entscheidend für die antikoagulatorische Wirksamkeit ist, jedoch deutlich weniger ausgeprägt als bei dem erfindungsgemäßen Heparin.

Daß die soeben beschriebenen Unterschiede bei den *in vivo*-Wirkungen von Enoxaparin und dem erfindungsgemäßen Heparin auftreten und der Grund dafür die unterschiedliche Bioverfügbarkeit ist, läßt sich durch einen Vergleich mit den bereits erwähnten und in Tabelle 1 angegebenen *in vitro*-Aktivitäten der drei Heparine belegen. Hier liegen die Verhältnisse ganz anders, wobei das erfindungsgemäße Heparin sowohl hinsichtlich der anti-FXa-Aktivität als auch hinsichtlich der Antithrombinaktivität die höchste Wirkung zeigt. Dieser Unterschied läßt nur den Schluß zu, daß der Einfluß auf das Ergebnis der Untersuchungen, die auf eine subkutane Verabreichung der drei Heparine an unterschiedliche Testpersonen zurückgehen, auf eine veränderte Verfügbarkeit der Wirksubstanzen im biologischen System, der Bioverfügbarkeit, im Unterschied zu den in *vitro*-Aktivitäten beruhen.

Die in den Figuren 3a bis 3b dargestellten Werte geben jedoch nicht die massenspezifische Aktivität eines jeden einzelnen Heparins an, da die spezifischen Aktivitäten der drei Heparine sich voneinander unterscheiden und jeweils 9.000 i.E. anti-FXa von dem erfindungsgemäßen Heparin, von Liquemin® und Enoxaparin den einzelnen Testpersonen injiziert worden waren. Daher wurden auch die dosisstandardisierten Werte für AUC₀₋₂₄ₕ berechnet, d.h. der Wert für AUC₀₋₂₄ₕ/mg des jeweils verabreichten Heparins. Die jeweils verabreichten Dosen betrugen 51,5 mg des erfindungsgemäßen Heparins, 56,5 mg Liquemin® und 90 mg Enoxaparin. Der Vergleich der massenspezifischen AUC-Werte, dargestellt in den Figuren 4a bis 4c, zeigt deutlich, daß das erfindungsgemäße Heparin nicht nur über eine höhere Bioverfügbarkeit als Liquemin® verfügt, sondern auch über ein wesentlich höheres Potential als Enoxaparin.

Beim Vergleich der Cₘₐₓ-Werte der drei untersuchten Heparine, die auf einer Verlängerung der Koagulationszeit und der Inhibierung beruhen, ist festzustellen, daß die Werte für das erfindungsgemäße Heparin wie die AUC₀₋₂₄ₕ-Werte höher als die von Liquemin® sind. Jedoch waren die Werte höchstens 50 % (anti-FXa-Aktivität) höher, wohingegen der entsprechende AUC₀₋₂₄ₕ-Wert doppelt so hoch war. Diese Diskrepanz beruht auf Unterschieden zwischen dem erfindungsgemäßen Heparin und Liquemin® in einigen pharmacokinetischen Parametern. Darüberhinaus kann die Überlegenheit des erfindungsgemäßen Heparins gegenüber Liquemin®, die bei den AUC₀₋₂₄ₕ-Wert ausgeprägter als in dem cₘₐₓ-Wert ist, von Vorteil sein, da im umgekehrten Fall das Risiko von Blutungen höher sein könnte. Durch Verringerung der Dosis kann cₘₐₓ unter Beibehaltung eines höheren AUC₀₋₂₄ₕ-Wertes als der von Liquemin® herabgesetzt werden.

Analog ist die Diskrepanz zwischen dem cₘₐₓ von Enoxaprin und Liquemin® in dem anti-Xa- und Antithrombinassay auch geringer als zwischen den AUC₀₋₂₄ₕ-Werten. Trotz des höheren AUC₀₋₂₄ₕ-Wertes ist der cₘₐₓ-Wert von Enaxoperin beim APTT-Test sogar 25 % niedriger als der von Liquemin®. Der Vergleich der pharmacokinetischen Parameter cₘₐₓ, t1/2_{Invasion}[min] und t1/2_{Elimination}[min] zeigt, daß das erfindungsgemäße Heparin weder mit UFH noch mit NMH vergleichbar ist. In Abhängigkeit von dem Parameter unterscheidet sich das erfindungsgemäße Heparin entweder deutlich von Liquemin® und Enoxaparin (z.B,. t1/2_{Invasion}) oder nur von Liquemin® (z.B. tₘₐₓ) oder nur von Enoxaparin (z.B. t1/2_{Elimination}).

Hinsichtlich der Zeit des Aktivitätsmaximums ist lediglich auszuführen, daß das erfindungsgemäße Heparin einen tₘₐₓ-Wert besitzt, der dem von Enoxaparin in etwa entspricht und sich nur gering von dem von Liquemin® unterscheidet. Es waren keine signifikanten Unterschiede bei den drei verschiedenen Assays für die drei untersuchten Heparine zu beobachten.

Der Wert für t1/2_{Invasion} ist für das erfindungsgemäße Heparin in dem APTT-Test und dem anti-FXa-Assay signifikant länger als für Liquemin®, jedoch nicht signifikant länger in dem anti-Flla-Assay. Außerdem ist diese Zeit länger als die von Enoxaparin in diesen beiden Assays. In dem anti-Flla-Assay besitzt Enoxaparin jedoch den größten t1/2_{Invasion}-Wert, was signifikant ist. Im Gegensatz zu anderen zeitbezogenen Werten unterscheidet sich der Wert für t1/2_{Elimination} des erfindungsgemäßen Heparins nicht signifikant von dem von Liquemin®. Der t1/2_{Elimination}-Wert von Enoxaparin ist in dem APTT-Test und dem anti-FXa-Assay doppelt so hoch wie der des erfindungsgemäßen Heparins und von Liquemin. In dem anti-FIIa-Assay besitzt Enoxaparin jedoch den kleinsten Wert für t1/2_{Elimination}. Die anti-FIIa-Aktivität des erfindungsgemäßen Heparins wird daher am langsamsten eliminiert, was aufgrund der Bedeutung der Antithrombinaktivität für die antikoagulatorische Wirksamkeit als Vorteil gegenüber den anderen beiden Heparinen anzusehen ist.

Die vorstehend diskutierten Werte sind nachfolgend in der Tabelle 3 für die drei untersuchten Heparine in den drei Assays zusammengefaßt.

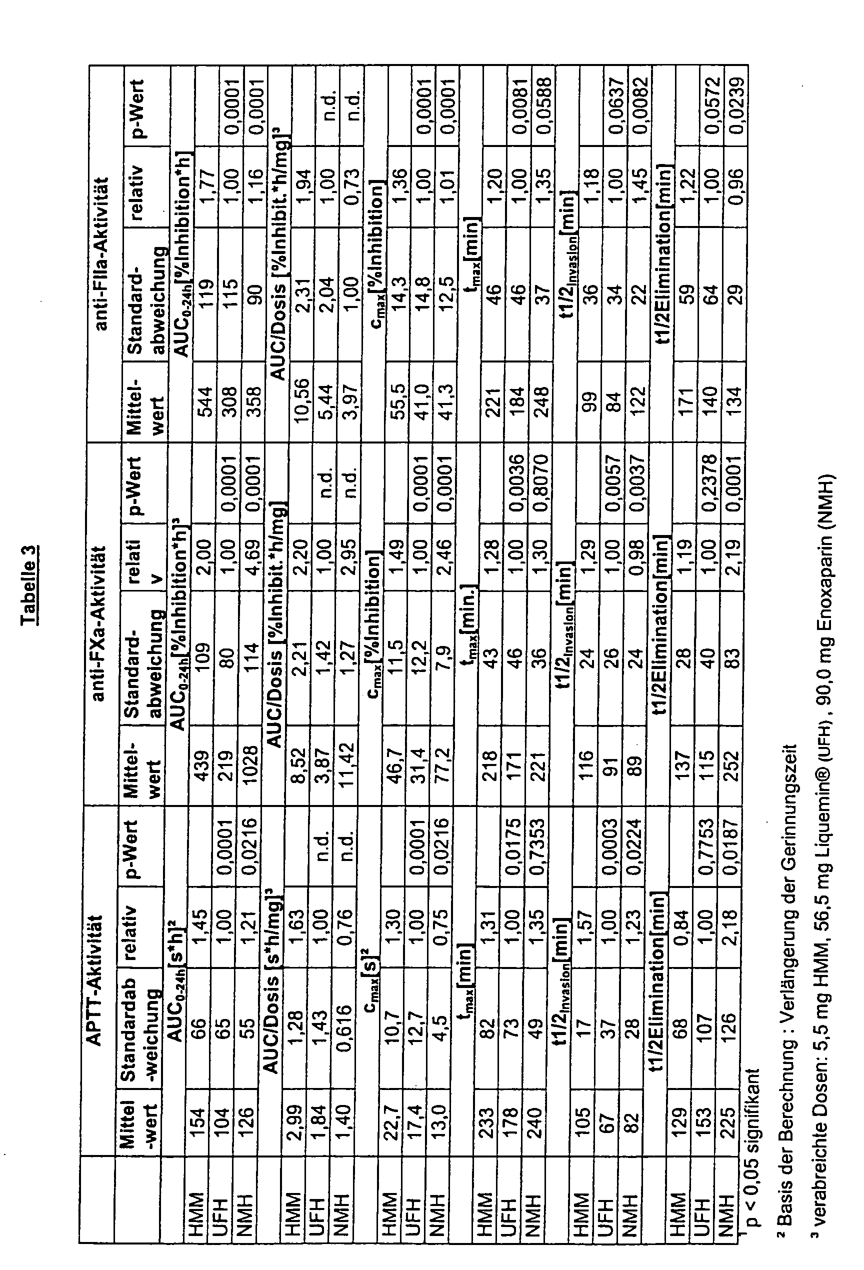

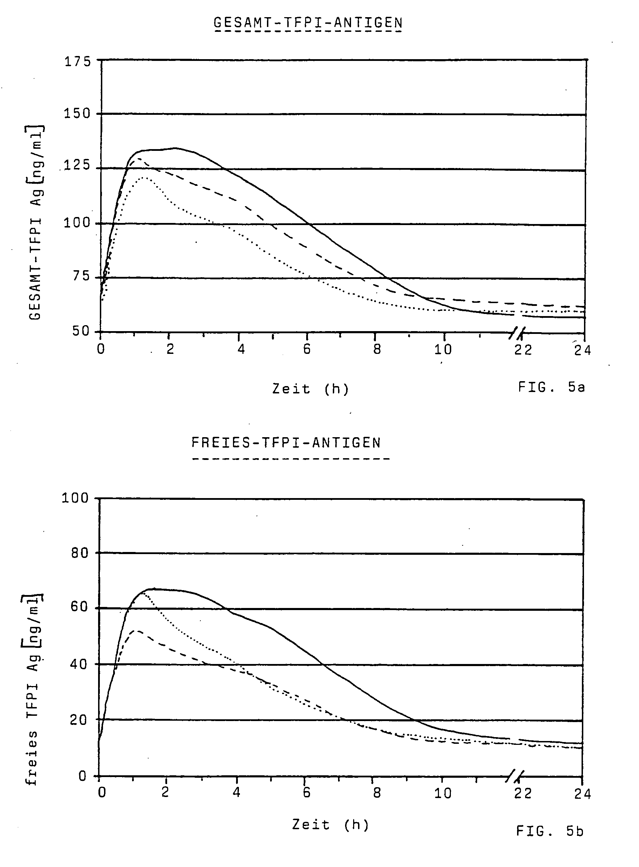

Bei der Betrachtung und dem Vergleich der Gesamt-TFPI-Antigenkonzentrationen und der Konzentrationen der freien TFPI-Antigens nach der Verabreichung der drei verschiedenen Heparine zeigt sich, daß die Konzentration des Gesamt-TFPI-Antigens sich nach einer Stunde auf das 2,0 bis 2,5-fache des Anfangswerts erhöht und die Konzentration des freien TFPI-Antigens auf das 6,7 bis 7,9-fache der Anfangskonzentration. Aus den Figuren 5a und 5b ist dabei ersichtlich, daß die Maximalwerte nach 1,5 bis 2 Stunden gefunden werden, so daß tₘₐₓ bedeutend kürzer als in den drei bisher durchgeführten Assays ist. Dies zeigt, daß die hepariniinduzierte TFPI-Freisetzung nicht mit diesen traditionellen Koagulationsparametern korreliert.

Aus Figur 5a und Figur 5b ist dabei weiterhin zu erkennen, daß die Wirkung des erfindungsgemäßen Heparins der von Liquemin® und Enoxaparin deutlich verschieden ist. Beide in diesem Zusammenhang untersuchten Werte verbleiben nach einer Verabreichung des erfindungsgemäßen Heparins signifikant länger auf einem hohen Niveau als nach Verabreichung von einem der Vergleichsheparine.

Auch diese Wirkung des erfindungsgemäßen Heparins ist sehr überraschend und von besonderer Bedeutung für die vorliegende Erfindung, da insbesondere bei dem freien TFPI-Antigen davon ausgegangen wird, daß es eine bedeutende Rolle bei der Gerinnung spielt.

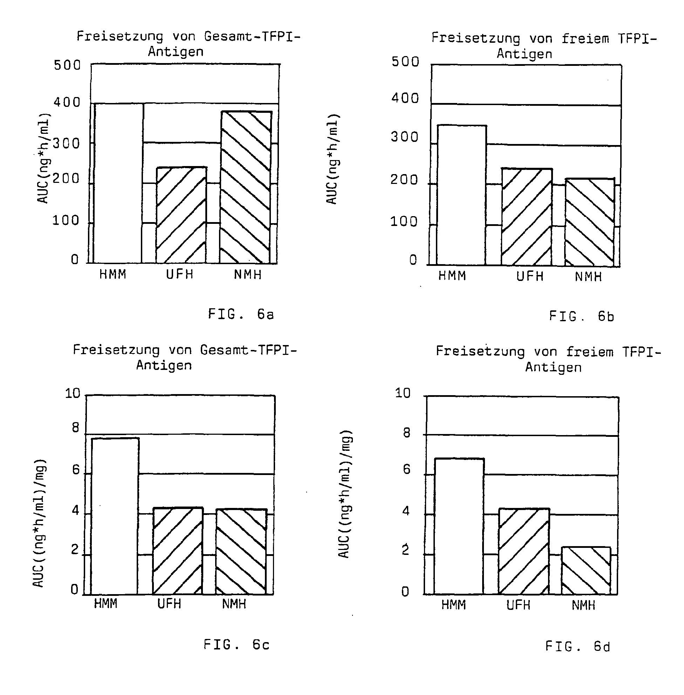

Beim Vergleich der entsprechenden AUC₀₋₂₄ₕ-Werte auf der Basis der Konzentrationen an freigesetztem TFPI (für Gesamt-TFPI-Antigen und freies TFPI-Antigen) wird deutlich, daß das erfindungsgemäße Heparin und Enoxaparin die Plasmakonzentrationen des Gesamt-TFPI-Antigens ungefähr in gleicher Weise erhöhen und Liquemin® klar überlegen sind, wie es Figur 6a zu entnehmen ist.

Da jedoch die Konzentration des freien TFPI-Antigens der für die Gerinnung kritische Parameter ist, wenn es um den Beitrag von TFPI zu der Aktivität von Heparin geht, müßten die AUC₀₋₂₄ₕ₋Werte für die Konzentration des freien TFPI-Antigens verglichen werden. Aus Figur 6b wird dabei ersichtlich, daß das erfindungsgemäße Heparin sich als am wirksamsten zeigt.

Vergleicht man nun die dosisnormierten AUC₀₋₂₄ₕ-Werte auf der Basis der Konzentrationen von freigesetztem TFPI so ergibt sich das in den Figuren 6c und 6d dargestellte Ergebnis. Sowohl für die Konzentration an Gesamt-TFPI-Antigen als auch die Konzentration an freiem TFPI-Antigen ist für das erfindungsgemäße Heparin am größten. Es dabei festzustellen, daß das erfindungsgemäße Heparin bei zwei entscheidenden Parametern für die Gerinnung, der Antithrombinaktivität und der Freisetzung von freiem TFPI-Antigen, den bekannten Heparinen eindeutig und zudem auch äußerst überraschend stark überlegen ist.

Abschließend ist zu erwähnen, daß diese Ergebnisse durch einen Vergleich des erfindungsgemäßen Heparins mit Enoxaparin im Tierversuch bestätigt werden. Dieser Tierversuch wurde an Kaninchen durchgeführt und zeigte eindeutig, die bessere therapeutische Wirkung des erfindungsgemäßen Heparins bei der Vorbeugung und der Behandlung thromboembolytischer Prozesse im Vergleich mit niedermolekularen Heparinen. Es zeigte sich dabei außerdem eine geringere Gefahr hinsichtlich des Auftretens von Blutungen nach der Verabreichung von dem erfindungsgemäßen Heparin im Vergleich zu Enoxaparin.

Es ist aufgrund der vorstehend beschriebenen Untersuchungsergebnisse ferner bevorzugt das erfindungsgemäße Heparin mit mittlerer Molmasse auch zur Therapie eines akuten Myokardinfarkts und instabiler Angina Pectoris sowie zur Gerinnungshemmung in extra korporalen Kreisläufen einzusetzen.

Es ist hervorzuheben, daß dieüberraschenden Eigenschaften des erfindungsgemäßen Heparins mit mittlerer Molmasse sich insbesondere beim APTT-Test zeigen, der als Maßstab bei der Heparintherapie grundlegend ist. Die Gerinnungszeit im APTT-Test ist, im Vergleich zu den untersuchten Heparinen Enoxaparin und Liquemin®, signifikant verlängert. Dabei ist auch die besonders hohe Milligrammaktivität des erfindungsgemäßen Heparins von Bedeutung, die ebenfalls völlig überraschend ist. Zusammen mit einem deutlich vermindertem Blutungsrisiko, das im Tierversuch eindrucksvoll die Überlegenheit des erfindungsgemäßen Heparins mit mittlerer Molmasse zeigt, eröffnen sich durch die vorliegende Erfindung völlig neue Perspektiven bei der Heparintherapie.

Hinsichtlich Wirkung und Verträglichkeit wurde das erfindungsgemäße Heparin mittlerer Molmasse auch im Tierversuch mit einem niedermolekularen Heparin, Enoxaparin, verglichen. Dies geschah im Rahmen eines modifizierten Thrombosemodells nach S. Wessler ("rabbit stasis model", "Thrombosis in the presence of vascular stasis", Am J. Med., 33:649, 1959), wobei die experimentell induzierte Thrombose aus der Kombination von Hypercoagulabilität und Venostase resultiert.

Zur Erfassung der Blutungsneigung dienten zwei vielfach bewährte und etablierte Verfahren, das so genannte "rabbit blood loss model" (Auszählung der Erythrozyten in der Spülflüssigkeit nach definierter Verletzung) und die "rabbit template bleeding time", bei der die Blutungszeit bestimmt wird (Fareed, J., Walenga J.M. et al., Studies on the antithrombotic effects and pharmacocinetics of heparin fractions and fragments, Sem. Thromb. Hemost. 11, 56-74, 1985).

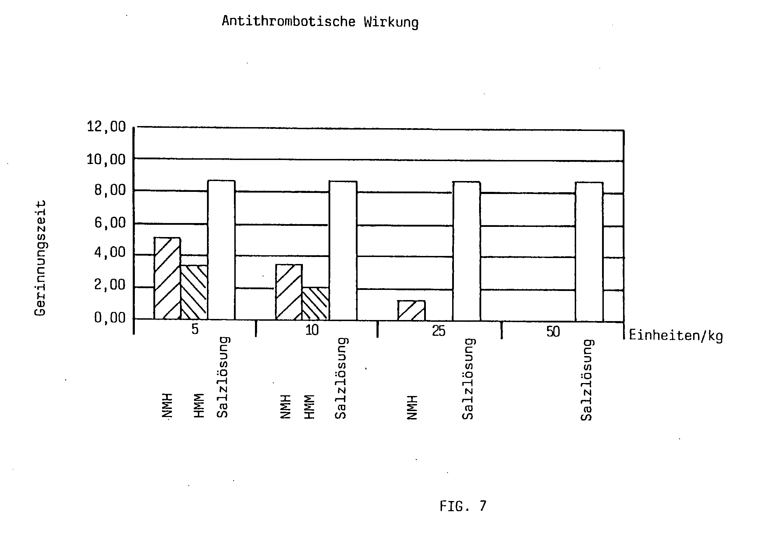

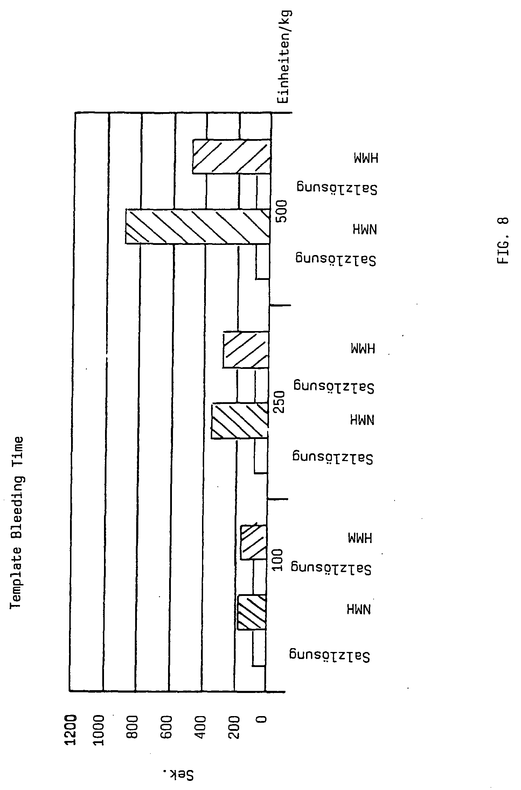

Die Figuren 7 und 8 belegen die eindrucksvolle, konsistente Erhöhung des "therapeutischen Index" des erfindungsgemäßen Heparins mit mittlerer Molmasse im Vergleich zu NMH, was nicht nur auf der gesteigerten antithrombotischen Wirksamkeit beruht, sondern auch besonders auf der signifikanten Herabsetzung der Blutungsneigung.

Figur 7 zeigt nochmals einen Vergleich der antithrombotischen Wirksamkeit bei intravenöser Gabe des als NMH Enoxaprins mit dem HMM der vorliegenden Erfindung. Aus der Figur ist dabei ersichtlich, daß der Gerinnungswert, der ein Maß für die Anzahl und Größe der gebildeten Blutgerinsel darstellt, bei dem erfindungsgemäßen Heparin deutlich geringer als bei Enoxaparin. Salzlösung wurde jeweils als Nullvergleich verwendet. Es zeigt sich dabei auch, daß die Gerinnung erst bei erhöhter Dosis, vorliegend bei 50 Einheiten/kg Körpergewicht, auch von Enoxaparin vollständig unterdrückt wird.

Figur 8 zeigt einen Vergleich der Blutungszeit auf intravenöse Verabreichung von Enoxaparin, HMM bzw. Salzlösung als Nullvergleich oder Standard. Es zeigt sich dabei gerade bei höheren Dosierungen, daß das erfindungsgemäße Heparin mit mittlerer Molmasse gegenüber dem Enoxaparin ein weitaus größeres Potential aufweist, da aus dem Tierversuch die absolut geringere Blutungsneigung deutlich wird.

Faßt man nun diese Ergebnisse zusammen, so läßt sich für das erfindungsgemäße HMM ein so genannter therapeutischer Index in Bezug auf das NMH Enoxaparin ermitteln. Dabei werden die Daten für die Wirksamkeit, d.h. die antithrombotische Wirkung, und die Daten für die Blutungsneigung mittels Varianzanalyse und linearer Regression ausgewertet und korreliert. Das Ergebnis ergibt einen therapeutischen Index von 2,24 für das erfindungsgemäße Heparin mit mittlerer Molmasse in Bezug auf Enoxaparin. Anders ausgedrückt das erfindungsgemäße Heparin mittlerer Molmasse besitzt den 2,24-fachen Anwendungswert in Bezug auf das Enoxaparin. Diese Ergebnisse sind für den Fachmann vollkommen überraschend und ermöglichen eine signifikante Verbesserung der Heparintherapie ca. 60 Jahre nach dem ersten Einsatz von unfraktionierten Heparinen und etwa 20 Jahre nach dem ersten Einsatz der niedermolekularen Heparine. Die vorliegende Erfindung befriedigt daher ein langanhaltendes Bedürfnis in der Fachwelt.

Durch die vorliegende Erfindung wird im Vergleich zum Stand der Technik ein ausdrücklich besseres Heparin zur Verfügung gestellt, das sich durch einen höheren therapeutischen Index auszeichnet, der auf einem günstigeren Verhältnis von Wirkung und Verträglichkeit beruht. Für die Klinik läßt sich dabei eine größere Reichweite bei der Prophylaxe und Therapie prognostizieren: Das erfindungsgemäße Heparin mittlerer Molmasse wird relativ höher dosiert werden können als die bekannten Heparine und damit zur einer noch nicht genau vorhersagbaren weiteren Verbesserung im Management thromboembolytischer Erkrankungen beitragen.

Dies ist insbesondere deshalb unerwartet, weil es aus klinischen Parametern nicht herleitbar ist.

Heparin,
dadurch gekennzeichnet,
daß es eine mittlere Molmasse von 10 bis 11,5 kd aufweist, insbesondere eine mittlere Molmasse von 10,5 kd.

Heparin , erhältlich durch kontrollierte
Depolymerisierung von unfraktioniertem Heparin mit salpetriger Säure und nachfolgender Molekularfiltration.

Verwendung eines Heparins . zur Herstellung eines Arzneimittels zur Prophylaxe und Therapie thromboembolytischer Prozesse, Therapie eines akuten Myokardinfarkts, instabiler Angina pectoris sowie zur Gerinnungshemmung in extrakorporalen Kreisläufen.

Verwendung
dadurch gekennzeichnet,
daß das Arzneimittel weiterhin pharmazeutisch verträgliche Füllund/oder Hilfsstoffe enthält.

Verwendung
dadurch gekennzeichnet,
daß das Arzneimittel zur parenteralen Verabreichung formuliert ist.

## Patentansprüche

1. Heparin mit einer mittleren Molmasse von 10 bis 11,5 kd mit einer anti-Xa-Aktivität von 174,8 und einer anti-IIa-Aktivität von 170,0, jeweils bestimmt nach dem 1. internationalen Standard für niedermolekulare Heparine.
